# EUROPEAN PATENT APPLICATION

(11) **EP 4 641 497 A1**
(43) Date of publication of application: **29.10.2025**
(21) Application number: 24172615.7
(22) Date of filing: 26.04.2024
(51) Int. Cl.: G06T 7/246, A61B 34/10

(54) **SYSTEM AND METHOD FOR DETECTING SUBJECT MOVEMENT DURING IMAGING PROCEDURE**

(71) Applicant: Koninklijke Philips N.V., 5656 AG Eindhoven (NL)
(72) Inventor: FRERKING, Lena Christina, Eindhoven (NL); SENEGAS, Julien Thomas, Eindhoven (NL)
(74) Representative: Philips Intellectual Property & Standards

(57) **Abstract**

A system and method for detecting movement of a subject during an imaging procedure that involves moving the subject on a subject support relative to a scanning gantry. A sequence of 2D image frames of the subject, acquired during movement of the subject support before a diagnostic scan of the imaging procedure, and support motion data, representative of motion of the subject support during the acquisition of the first sequence of 2D image frames, are received and processed to generate an averaged depth map of the subject. One or more further 2D image frames of the subject, acquired prior to and/or during the diagnostic scan, are received and processed using the depth map to identify any movement of the subject relative to the subject support.

## Description

### FIELD OF THE INVENTION

The invention relates to the field of medical imaging, and in particular, to motion detection during medical imaging procedures.

### BACKGROUND OF THE INVENTION

Motion of a subject during a CT or MRI examination can lead to motion artifacts if the motion occurs in an area that includes a current scan plane.

Using existing methods for detecting subject movement, it is often not possible to determine that movement has occurred within the scan plane before the examination has been completed. In other words, by the time a determination that movement occurred within the scan plane has been made, the subject has generally left the subject support of the scanning device. This means that it is necessary to repeat the entire imaging procedure (including a localizer scan) in order to re-image planes in which motion occurred.

There is therefore a need for improved motion detection during imaging procedures.

### SUMMARY OF THE INVENTION

The invention is defined by the claims.

According to examples in accordance with an aspect of the invention, there is provided a processing system for detecting movement of a subject during an imaging procedure, wherein the imaging procedure involves moving a subject support relative to a scanning gantry of a scanner, the processing system being configured to: receive, from a camera, a first sequence of 2D image frames of the subject acquired by the camera during a period of motion of the subject support prior to a diagnostic scan of the imaging procedure; receive support motion data representative of motion of the subject support during the acquisition of the first sequence of 2D image frames; process the first sequence of 2D image frames and the support motion data to generate a depth map for the subject, wherein the generated depth map provides an average depth value for each of a plurality of points on the surface of the subject, adjusted for subject support position; receive, from the camera, one or more further 2D image frames of the subject acquired by the camera; and process the depth map and the one or more further 2D image frames to identify any movement of the subject relative to the subject support.

The inventors have recognized that motion of the subject support prior to a diagnostic scan (e.g. during a localizer scan, also known as a surview scan) may be used to generate a depth map that is robust with respect to noise and stable from frame to frame, and that such a depth map enables motion of the subject relative to the subject support to be identified.

The computation of a robust and stable depth map is relatively time-consuming, such that if the depth map is computed based on data acquired during a diagnostic scan (i.e. a scan for which the acquired imaging data is intended to be analyzed for diagnostic purposes), the generation of the depth map can take longer than the remaining time of the diagnostic scan. By computing the depth map based on data acquired prior to the diagnostic scan, for example, during the localizer scan, the depth map may be available for identifying subject motion before the start of the diagnostic scan.

The inventors have recognized that a depth map that combines information from several subject support positions is suitable for identifying motion in a subject prior to and/or during subsequent scans of the imaging procedure (e.g. diagnostic scans), including during times at which the subject support is not moving, as such a depth map can be virtually shifted according to the subject support position for a given frame.

Further, by using image frames from the same camera both to generate the depth map and to identify motion, errors in identifying correspondences between the depth map and the image frame(s) used to identify motion are reduced.

Identifying any movement of the subject relative to the subject support may comprise identifying a location of any movement of the subject relative to the subject support and/or identifying an amount of movement of the subject relative to the subject support (i.e. identifying a movement of the subject may comprise identifying a property of the movement).

The imaging procedure may, for example, be a CT, MRI, PET, PET-CT or SPECT procedure.

In some examples, the processing system is configured to generate the depth map by: processing the first sequence of 2D image frames and the support motion data to determine a set of 3D coordinates for each 2D image frame in the first sequence; processing the first sequence of 2D image frames and the support motion data to translate each set of 3D coordinates to a same reference position; processing each translated set of 3D coordinates to determine an average set of 3D coordinates; and processing the average set of 3D coordinates to generate the depth map.

In some examples, the first sequence of 2D image frames comprises at least two 2D image frames that capture different positions of the subject support, wherein the different positions of the subject support captured by the first sequence of 2D image frames encompass a predicted range of positions of the subject support during the diagnostic scan of the imaging procedure.

This improves a likelihood that the depth map is valid for all subject support positions in the diagnostic scan. For instance, the different positions captured by the first sequence of 2D image frames may include at least a position of the subject support outside the predicted range on one side of the predicted range and a position outside the predicted range on the other side of the predicted range. Preferably, the positions of the subject support captured by the first sequence of 2D image frames comprises a plurality of positions distributed throughout the predicted range of positions of the subject support during the diagnostic scan. For instance, the first sequence of 2D image frames may comprise an image frame captured every 5 mm from beginning to end of the predicted range of positions of the subject support during the diagnostic scan.

The greater the range of positions of the subject support captured in the first sequence of 2D image frames, the more robust the depth map generated using the first sequence.

In some examples, the first sequence of 2D image frames is acquired by the camera prior to or during a localizer scan of the subject.

A localizer scan (also known as a surview scan) is typically carried out during an imaging procedure in advance of the diagnostic scan in order to locate an anatomical target of the imaging procedure. Acquiring the first sequence of 2D image frames prior to or during the localizer scan allows the depth map to be generated before the start of the diagnostic scan.

In some examples, the first position of the subject support is a position at a first end of a path along which the subject support is moved during the localizer scan, and the second position of the subject support is a position at a second end of the path.

In other words, the first sequence of 2D image frames captures the full range over which the subject support is moved during the localizer scan.

In some examples, the processing system is further configured to process the depth map and the one or more further 2D image frames to identify a region of interest in each of the one or more further 2D image frames.

In some examples, the processing system is configured to identify any movement of the subject relative to the subject support by processing the depth map and the one or more further 2D image frames to identify any movement of the subject relative to the subject support within the region of interest.

In this way, the processing system may only detect movements of the subject that are considered likely to affect a clinical usefulness of imaging data acquired during the imaging procedure.

In some examples, the processing system is further configured to: generate an indication of a position of the region of interest in each of the one or more further 2D image frames; and control a display device to display the indication of the position of the region of interest.

For instance, the indication may be provided as an overlay in each of the one or more further 2D image frames.

Displaying the indication of the position of the region of interest provides guidance to a clinician carrying out the imaging procedure.

In some examples, the region of interest defines a position of a scan plane in each of the one or more further 2D image frames.

In some examples, the processing system is further configured to, in response to identifying a movement of the subject relative to the subject support: determine whether the identified movement satisfies one or more predetermined conditions; and in response to a determination that the identified movement satisfies the one or more predetermined conditions, control an output user interface to provide a user-perceptible output representative of the identified movement.

In this way, a clinician may be alerted to subject movement that may affect the imaging procedure. For instance, if movement is identified before a start of a diagnostic scan, this allows the clinician to delay the diagnostic scan until the subject has stopped moving. If movement is identified during a scan, it allows a clinician to re-scan the relevant region while the subject is still in position on the subject support.

In some examples, determining whether the identified movement satisfies one or more predetermined conditions may comprise determining a measure of movement for the identified movement, and determining whether the measure of movement satisfies one or more predetermined conditions (e.g. whether the measure of movement exceeds a predetermined threshold).

In some examples, the processing system is further configured to, in response to identifying a movement of the subject relative to the subject support: determine a measure of movement for the identified movement; in response to the measure of movement exceeding a predetermined threshold: receive, from the camera, a second sequence of 2D image frames of the subject acquired by the camera during motion of the subject support following the identified movement; process the second sequence of 2D image frames to generate an updated depth map for the subject; and for any further 2D image frame received after generating the updated depth map, process the updated depth map and the further 2D image frame to detect any further movement of the subject relative to the subject support.

Updating the depth map each time a sufficiently large movement of the subject is identified ensures that the depth map being used for motion detection is accurate.

In some examples, the processing system is further configured to repeat, at predetermined intervals during motion of the subject support, the steps of: receiving, from the camera, a further sequence of 2D image frames of the subject acquired by the camera during the motion of the subject support; and processing the further sequence of 2D image frames to generate an updated depth map for the subject, wherein the processing system is configured to, for any further 2D image frame received after generating the updated depth map, process the most-recently generated updated depth map and the further 2D image frame to identify any movement of the subject relative to the subject support.

In this way, a recently-generated depth map is always available for motion detection.

In some examples, the processing system is further configured to control the scanner based on whether or not movement of the subject relative to the subject support is identified.

For example, the processing system may control the scanner not to image the subject during a period in which movement of the subject relative to the subject support is identified, or during a period in which movement of the subject relative to the subject support is identified and satisfies one or more predetermined conditions.

There is also provided a system for detecting movement of a subject during an imaging procedure, wherein the imaging procedure involves moving a subject support relative to a scanning gantry of a scanner, the system comprising: a camera configured to acquire 2D image frames of the subject during the imaging procedure; and the processing system described above.

According to examples in accordance with another aspect of the invention, there is provided a computer-implemented method for detecting movement of a subject during an imaging procedure, wherein the imaging procedure involves moving a subject support relative to a scanning gantry of a scanner, the computer-implemented method comprising: receiving, from a camera, a first sequence of 2D image frames of the subject acquired by the camera during period of motion of the subject support prior to a diagnostic scan of the imaging procedure; receiving support motion data representative of motion of the subject support during the acquisition of the first sequence of 2D image frames; processing the first sequence of 2D image frames and the support motion data to generate a depth map for the subject, wherein the generated depth map provides an average depth value for each of a plurality of points on the surface of the subject, adjusted for subject support position; receiving, from the camera, one or more further 2D image frames of the subject acquired by the camera; and processing the depth map and the one or more further 2D image frames to identify any movement of the subject relative to the subject support.

There is also provided a computer program product comprising computer program code means which, when executed on a computing device having a processing system, cause the processing system to perform all of the steps of the method described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a system for detecting movement of a subject during an imaging procedure, according to an embodiment of the invention; and
Fig. 2 illustrates a computer-implemented method for detecting movement of a subject during an imaging procedure, according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system and method for detecting movement of a subject during an imaging procedure that involves moving the subject on a subject support relative to a scanning gantry. A sequence of 2D image frames of the subject, acquired during movement of the subject support before a diagnostic scan of the imaging procedure, and support motion data, representative of motion of the subject support during the acquisition of the first sequence of 2D image frames, are received and processed to generate an averaged depth map of the subject. One or more further 2D image frames of the subject, acquired prior to and/or during the diagnostic scan, are received and processed using the depth map to identify any movement of the subject relative to the subject support.

Embodiments are at least partly based on the realization that movement of the subject support during the localizer scan of an imaging procedure enables the generation of a depth map that is averaged over image frames at different table positions, resulting in a depth map that is robust and stable, and that such a depth map may be generated before the start of a diagnostic scan of the imaging procedure, allowing movement of the subject relative to the subject support to be identified throughout the diagnostic scan, and even before the start of the diagnostic scan.

Illustrative embodiments may, for example be employed in CT systems, conventional radiography (X-ray) systems, fluoroscopic systems, interventional X-ray systems, MRI systems, MR-Linac systems, PET systems, PET-CT systems and/or SPECT systems.

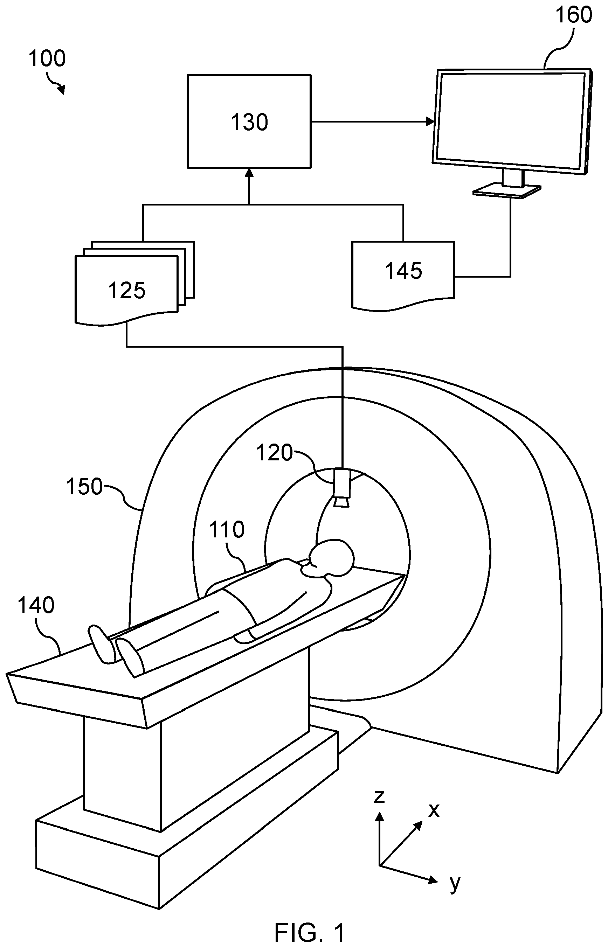

Fig. 1 illustrates a system 100 for detecting movement of a subject 110 during an imaging procedure, according to an embodiment of the invention. The system 100 comprises a camera 120 and a processing system 130. The processing system is, itself, an embodiment of the invention.

The system 100 may be used to detect movement of a subject during any imaging procedure that involves moving a subject support 140 (also known as a "support table" or "patient couch") relative to a scanning gantry 150 of a scanner, thereby moving a subject on the subject support relative to a scanning plane of the scanner. The subject support is configured to be movable in a support movement direction x.

In Fig. 1, the scanner is a computed tomography (CT) scanner; in other examples, the scanner may be a conventional radiography (X-ray) scanner, a fluoroscopic scanner, a scanner of an interventional X-ray system, a magnetic resonance imaging (MRI) scanner, an MR-Linac scanner, a position emission tomography (PET) scanner, a PET-CT scanner, or a single-photon emission computed tomography (SPECT) scanner.

The camera 120 is an optical camera configured to acquire 2D image frames 125 of the subject 110 during the imaging procedure. In Fig. 1, the camera is provided at the entry to the scanning gantry 150, such that the optical axis of the camera is perpendicular to the support movement direction x. However, the camera may be provided in any fixed position relative to the scanning gantry that is suitable for acquiring images of the subject as the subject support 140 is moved relative to the scanning gantry. The camera 120 may acquire 2D image frames of the subject throughout the imaging procedure (i.e. the camera may acquire a video of the imaging procedure).

As used herein, the term "imaging data" refers to imaging data acquired by the scanner used to carry out the imaging procedure, the collection of which is the purpose of the imaging procedure. For instance, the imaging data is CT imaging data in the case of a CT imaging procedure, and MRI data in the case of an MRI procedure. The term "2D image frames" is used herein to refer to optical images acquired by the camera 120.

The processing system 130 is configured to receive, from the camera 120, the 2D image frames 125 of the subject acquired by the camera. The processing system 130 may receive each 2D image frame as it is acquired by the camera.

The 2D image frames 125 acquired by the camera 120 and received by the processing system 130 include a first sequence of 2D image frames of the subject acquired by the camera during period of motion of the subject support prior to a diagnostic scan of the imaging procedure. A diagnostic scan is a scan carried out in order to achieve the primary purpose of the imaging procedure (e.g. to acquire imaging data of a target anatomical feature for diagnostic purposes).

In some examples, the 2D image frames 125 may be acquired by the camera prior to or during a localizer scan of the subject. A localizer scan (also known as a surview scan or scout scan) is a scan performed during an imaging procedure in advance of the diagnostic scan, in order to locate a target region to be imaged during the diagnostic scan (i.e. a region containing a target anatomical feature).

In some types of imaging procedure (e.g. CT imaging procedures, the subject is moved on the subject support, relative to the scanning gantry, during a localizer scan. In such cases, the subject is typically moved on the subject support, relative to the scanning gantry, over a greater distance than during a diagnostic scan that follows the localizer scan, allowing 2D image frames to be acquired over at least the range of subject support positions of the diagnostic scan.

In other types of imaging procedure (e.g. MRI procedures), the subject support may be stationary during the localizer and diagnostic scan; however, the subject support is moved relative to the scanning gantry prior to the localizer scan in order to position the subject (e.g. to move the subject into an MRI bore in the case of an MRI procedure). Image frames acquired during this period of movement of the subject support are similarly suitable for generating a depth map, as described in more detail below.

The first sequence of 2D image frames comprises at least two 2D image frames that capture different positions of the subject support 140 relative to the scanning gantry 150. Preferably, the range of positions of the subject support captured in the first sequence of 2D image frames encompasses at least all positions through which the subject support is to be moved during the diagnostic scan. For instance, the first sequence of 2D image frames may capture positions of the subject support distributed throughout the predicted range of subject support positions during the diagnostic scan, for example, positions at regular intervals throughout the predicted range (e.g. at intervals of 2-20 mm).

In some examples, the range of positions of the subject support captured in the first sequence of 2D image frames (i.e. a greatest distance between the positions captured) may be greater than the predicted range of positions of the subject support during the diagnostic scan; for example, the different positions captured by the first sequence of 2D image frames may include at least one position of the subject support outside the predicted range on one side of the predicted range and at least one position outside the predicted range on the other side of the predicted range. In some examples, the first sequence of 2D image frames may include at least a first image frame, capturing the subject 110 when the subject support 140 is at a first end of the path along which the subject support is moved during the localizer scan, and a second image frame, capturing the subject when the subject support is at a second, opposite end of the path.

The processing system 130 is further configured to receive support motion data 145 representative of motion of the subject support 140 during the acquisition of the first sequence of 2D image frames. The support motion data may be any data that enables a speed of the subject support during the acquisition of the first sequence of 2D image frames to be determined.

The speed of the subject support during the imaging procedure is typically defined by an operator of the imaging procedure. The support motion data 145 may therefore comprise parameters of an exam card for the imaging procedure; for instance, collimation, pitch and rotation, where "pitch" is a distance moved by the subject support relative to the collimation for a 360° rotation of the scanning gantry, and "rotation" is a time taken for a 360° rotation of the scanning gantry. In some examples, the support motion data may simply comprise the speed of the subject support, rather than parameters from which the speed is determined.

In Fig. 1, the subject support motion data 145 is received directly from a user input device 160 into which the operator of the imaging procedure inputs parameters for the imaging procedure. Alternatively, the subject support motion data may be received from a memory unit or from a controller configured to control motion of the subject support.

Having received the first sequence of 2D image frames and the support motion data 145, the processing system 130 is configured to process the first sequence of 2D image frames and the support motion data to generate a depth map for the subject 110 that provides an average depth value for each of a plurality of points on the surface of the subject (adjusted for subject support position).

The inventors have recognized that 2D image frames acquired, by a camera having a fixed position relative to the scanning gantry, during motion of the subject support relative to the scanning gantry enable the use of the stereo effect to generate a depth map of the subject. In particular, the speed of the subject support (determined from the support motion data) and the time between the acquisition of two image frames (i.e., in the case of successive image frames, the frame rate of the camera) allow the distance moved by the subject support between the acquisition of the two image frames to be determined. By averaging the depth map over a plurality of subject support positions (and, in particular, over a large range of subject support positions), a more robust depth map may be generated.

For instance, the processing system 130 may generate the depth map by processing the first sequence of 2D image frames and the support motion data 145 to determine a set of 3D coordinates for each 2D image frame in the first sequence. The set of 3D coordinates may be determined for each 2D image frame by, for example, comparing the distance moved by the subject support between the acquisition of the image frame and at least one other image frame with the difference in the position, in the support movement direction x, of the subject within the image frame and within the at least one other image frame. The intrinsic and extrinsic calibration parameters of the camera 120 may be used in the determination of the set of 3D coordinates for each 2D image frame.

The first sequence of 2D image frames and the support motion data may then be processed to translate each set of 3D coordinates to a same reference position (i.e. by using the support motion data to determine a distance from the reference position for each respective 2D image frame). Alternatively, the translated sets of 3D coordinates may be determined by first transforming each 2D image frame in the first sequence, using the intrinsic and extrinsic calibration parameters of the camera 120, and then processing the transformed 2D image frames and the support motion data to determine a translated set of 3D coordinates for each 2D image frame in the first sequence.

The translated sets of 3D coordinates may be used to determine an average set of 3D coordinates, which may be processed to generate the depth map. For instance, each 3D coordinate in the average set of 3D coordinates may be determined by determining the average of a group of 3D coordinates that each project to the same depth pixel in an image frame at the reference position. In some examples, outlier values in the translated sets of 3D coordinates may be excluded when determining the average set of 3D coordinates, in order to reduce an effect of occlusions in the average set of 3D coordinates. For example, for each 3D coordinate in the average set of 3D coordinates, any 3D coordinate that projects to the corresponding depth pixel in the image frame at the reference position having a depth value that is greater, by more than a predetermined margin, than the 3D coordinate in the group having the lowest depth value may be excluded from the group used to determine the average.

The depth map generated from the average set of 3D coordinates may be any suitable representation of the average set of 3D coordinates. For instance, the depth map may be a projection of the average set of 3D coordinates to an image frame (e.g. an image frame at the reference position), with each pixel of the image frame having a depth value, or a point cloud of the average set of 3D coordinates. In some examples, a depth map may be generated for each 2D image frame that is processed to detect motion; the depth map for a given 2D image frame may be generated based on the average set of 3D coordinates and the position of the subject support in the 2D image frame.

The generated depth map is used to identify movement of the subject 110 relative to the subject support 140. Identifying the movement of the subject may comprise, in addition to detecting the presence of movement of the subject, identifying a location (i.e. in 3D space) of the movement and/or identifying an amount of movement of the subject (e.g. in mm).

Movement of the subject 110 relative to the subject support 140 may be identified using one or more further 2D image frames. The one or more further 2D image frames includes at least one image frame acquired by the camera 120 after the acquisition of the first sequence of 2D image frames (i.e. the processing system 130 is configured to continue to receive 2D image frames 125 acquired by the camera after receiving the first sequence of 2D image frames). For instance, the one or more further 2D image frames may comprise a sequence of 2D image frames acquired throughout the remainder of the imaging procedure. In some examples, the one or more further 2D image frames may additionally include the first sequence of 2D image frames (i.e. after using the first sequence of 2D image frames to generate the depth map may be re-processed to identify any movement of the subject relative to the subject support that occurred during acquisition of the first sequence of 2D image frames).

The processing system 130 is configured to process the depth map and the one or more further 2D image frames to identify any movement of the subject relative to the subject support. Motion of the subject may be detected by comparing pixel intensities of each of the one or more further 2D image frames with another 2D image frame (e.g. a temporally adjacent image frame or an image frame belonging to the first sequence). A pixel shift may be computed for each pixel in each of the one or more further 2D image frames by determining a distance between the pixel and a corresponding pixel in the other 2D image frame, where the corresponding pixel is identified based on pixel intensity (e.g. the corresponding pixel may be a pixel with a most similar intensity within a corresponding region of the other 2D image frame). A non-zero pixel shift is indicative of motion.

A detected non-zero pixel shift may be converted to a motion vector (e.g. in mm) using the generated depth map and a depth map corresponding to the further 2D image frame in which the non-zero pixel shift is identified. The depth map corresponding to the further 2D image frame may be generated using the further 2D image frame (i.e. the depth map corresponding to the further 2D image frame is representative of the position of the subject support and the position of the subject in the further 2D image frame). As the subject support is able to move only in the support movement direction x, a detected movement in any other direction may be identified as movement of the subject relative to the subject support.

Motion of the subject (particularly in a direction parallel to the optical axis of the camera 120, i.e. direction z in Fig. 1) may also be detected in each of the one or more further 2D image frames by comparing a depth map corresponding to the further 2D image frame (i.e. generated using the further 2D image frame) with the generated depth map (where the generated depth map is generated by projecting the average set of 3D coordinates to an image frame having the same subject support position as the further 2D image frame)

Preferably, the processing system 130 is configured to receive and process each of the one or more further 2D image frames immediately after the image frame is acquired by the camera 120. As the depth map is generated based on image frames acquired before the diagnostic scan, this allows movement of the subject during the diagnostic scan (or between the generation of the depth map and the start of the diagnostic scan) to be identified in real time (or near real time).

Sometimes, movement of the subject 110 relative to the subject support 140 may occur during the period of movement of the subject support during which the first sequence of 2D image frames is acquired (e.g. during the localizer scan). In such cases, the first sequence of 2D image frames used to generate the depth map may include only image frames acquired after the movement of the subject occurred. Movement of the subject during this period may be identified by comparing pixel intensities between the acquired image frames. Alternatively, movement of the subject during this period may be identified after generation of the depth map, and a new depth map may be generated using only the 2D image frames acquired after the movement occurred.

In some examples, the processing system 130 may consider only a particular region of interest when identifying movement of the subject. The processing system may identify a region of interest in each of the one or more further 2D image frames by processing the depth map and the respective further 2D image frame. The processing system may then process the depth map and the one or more further 2D image frames to identify any movement of the subject relative to the subject support within the region of interest.

The region of interest may be a region for which movement of the subject relative to the subject support is likely to affect a clinical usefulness of imaging data acquired during the imaging procedure (e.g. movement that is likely to result in motion artifacts). For example, the region of interest define a scan plane of the scanner performing the imaging procedure, or a region including and surrounding the scan plane. In other examples, the region of interest may define a total region to be scanned during the diagnostic scan of the imaging procedure, or a region defining an anatomical area (e.g. torso, abdomen, etc.), for example an anatomical area containing a target anatomical feature of the imaging procedure.

Where the region of interest is defined with respect to the subject support or the subject (e.g. an anatomical area of the subject), the position of the region of interest within each of the one or more further 2D image frames may be determined by projecting the region of interest to the correct position in each further 2D image frame. During periods of movement of the subject support, a current speed of the subject support may be used to project the region of interest to the correct position. The current speed of the subject support may be obtained by receiving further support motion data representative of motion of the subject support during acquisition of the relevant further 2D image frame, or the current speed of the subject support may be determined from the one or more further 2D images (since the generated depth map enables a pixel shift between image frames to be translated to a distance measurement in 3D space (e.g. a shift in mm).

Where the region of interest has a fixed position relative to the scanning gantry (e.g. where the region of interest is defined by the scan plane), only the generated depth map is required to determine the position of the region of interest within each of the one or more further 2D image frames.

In some examples, identification of a region of interest in each of the one or more further 2D image frames may additionally or alternatively be used to generate and display an indication of the position of the region of interest, in order to provide guidance to an operator of the imaging procedure. The processing system 130 may be configured to generate an indication of a position of the region of interest in each of the one or more further 2D image frames, and control a display device to display the indication of the position of the region of interest.

The indication of the position of the region of interest within each of the one or more further 2D image frames may, for example, be provided as an overlay on the respective further 2D image frame. For instance, the display device may display a live video stream acquired by the camera 120, and each image frame in the video stream may be provided with an overlay indicating the position of the region of interest within the image frame.

In examples in which a region of interest is used both to define a region in which movement is identified and to display an indication, the same region of interest may be used both to identify movement and to display the indication. Alternatively, different regions of interest may be used for each: for instance, the processing system 130 may be configured to generate and display an indication of a first region of interest defining a scan plane, and to identify movement of the subject within a second region of interest. The second region of interest may, for example, form part of the first region of interest, overlap the first region of interest or be entirely outside the first region of interest.

In Fig. 1, the display device is the user input device 160 used to obtain the support motion data 145. However, as the skilled person will readily appreciate, in other examples, separate devices may be used to obtain the support motion data and to display the indication of the position of the region of interest.

In response to identifying a movement of the subject 110 relative to the subject support 140, the processing system 130 may be configured to alert a clinician to the identified movement. If the identified movement occurs between the localizer scan and the diagnostic scan, the clinician may decide that a new localizer scan is required in order to identify a new location of the target region before proceeding with the diagnostic scan, or decide to postpone the start of the diagnostic scan until no movement of the subject is detected. If the identified movement occurs during the diagnostic scan, the clinician may decide to repeat at least part of the diagnostic scan (e.g. to re-scan the region that was scanned during the detected movement).

The processing system 130 may be configured to alert a clinician to an identified movement by controlling an output user interface to provide a user-perceptible output representative of the identified movement. In Fig. 1, the output user interface is the user input/display device 160; however, as the skilled person will readily appreciate, any other suitable device may be used to provide the user-perceptible output. The user-perceptible output may comprise, for example, one or more of: an image, a light, a textual display, a sound and/or a vibration.

In some examples, the user-perceptible output may simply indicate that a movement of the subject 110 relative to the subject support 140 has been detected. In other examples, the user-perceptible output may provide additional information relating to the identified movement; for instance, the user-perceptible output may indicate an amount and/or location of movement. In some examples, the user-perceptible output may indicate which slices of imaging data acquired by the scanner are likely to have been affected by the movement of the subject (e.g. by adding an icon indicating motion on the potentially affected slices, presenting the affected slices to a clinician for review, or providing an indication (e.g. an overlay) of the position of the potentially affected slices on a surview image of the subject).

In some examples, the processing system 130 may control the output user interface to provide the user-perceptible output only in response to a determination that the identified movement satisfies one or more predetermined conditions. For instance, the processing system may control the output user interface to provide the user-perceptible output only in response to a determination that a measure of movement for the identified movement exceeds a predetermined movement threshold and/or that the identified movement occurred within a predefined region.

In some examples, the processing system 130 may be further configured to control the scanner performing the imaging procedure based on whether or not movement of the subject is identified. For instance, the processing system 130 may control the scanner not to start the diagnostic scan (e.g. not to start X-ray exposure in the case of a scanner that uses X-rays) in response to identifying movement of the subject relative to the subject support before the diagnostic scan has begun, or in response to an identified movement of the subject relative to the subject support before the diagnostic scan satisfying one or more predetermined conditions (e.g. a measure of movement for the identified movement exceeding a predetermined threshold and/or the identified movement occurring in a region of interest). The processing system may control the scanner to pause or stop scanning in response to identifying movement of the subject relative to the subject support during the diagnostic scan, or in response to an identified movement of the subject relative to the subject support during the diagnostic scan satisfying one or more predetermined conditions.

Once the subject 110 has moved relative to the subject support 140, a depth map that was generated based on 2D image frames acquired before the movement occurred may no longer be accurate. The processing system 130 may therefore be configured to update the depth map to improve a likelihood that the depth map remains accurate throughout the imaging procedure.

In some examples, the processing system 130 may be configured to update the depth map in response to identifying a movement of the subject 110 relative to the subject support 140 for which a measure of movement exceeds a predetermined threshold. The measure of movement may, for example, be the motion vector described above, or a magnitude of the motion vector. In some examples, the measure of movement is a motion index that represents the integral measured displacement within a given region of the subject over a time interval defined by the collimation length, the pitch and the speed of the subject support. As the skilled person will appreciate, the predetermined threshold will depend on the clinical application of the imaging procedure (e.g. on the anatomical target of the imaging procedure). The predetermined threshold may be defined via user input, or may be automatically selected from a look-up table according to the clinical application of the imaging procedure.

The processing system may update the depth map by receiving a second sequence of 2D image frames of the subject from the camera 120, the second sequence of 2D image frames having been acquired by the camera during motion of the subject support following the identified movement of the subject.

In order to make use of the stereo effect to determine 3D coordinates for the depth map, the second sequence of 2D image frames used to update the depth map must include image frames acquired during motion of the subject support 140. Therefore, if movement of the subject 110 is identified during a time at which the subject support is not moving relative to the scanning gantry, the depth map cannot be updated until the subject support is next moved. For instance, if movement of the subject relative to the subject support is identified between the localizer scan and the diagnostic scan, the second sequence of 2D image frames may be acquired at the start of the diagnostic scan (unless the localizer scan is repeated). If movement of the subject relative to the subject support is identified during a scanning step of a "step and shoot" scan, the second sequence of 2D image frames may be acquired the next time the subject support is moved.

The processing system 130 may process the second sequence of 2D image frames to generate an updated depth map for the subject. The updated depth map may be generated in the same way as described above with respect to the original depth map (i.e. an entirely new depth map may be generated based on the second sequence of 2D image frames). Alternatively, only regions of the depth map in which movement of the subject occurred may be updated. In other words, parts of the original depth map may be replaced with new depth values, determined using the second sequence of 2D image frames. This may reduce a time taken to generate the updated depth map.

The speed at which the subject support is moved during acquisition of the second sequence of 2D image frames is used to generate the updated depth map. The speed of the subject support during acquisition of the second sequence of 2D image frames may be obtained by receiving further support motion data representative of motion of the subject support during acquisition of the second sequence of 2D image frames, or by processing the second sequence of 2D image frames (or any other 2D image frames acquired at stage of the imaging procedure having a same subject support speed) to determine the speed of the subject support. A pixel shift between 2D image frames in the second sequence may be translated to a distance measurement in 3D space (e.g. a shift in mm) using the previously generated depth map.

Having generated the updated depth map, any further 2D image frame received once the updated depth map has been generated may be processed using the updated depth map to detect a further movement of the subject 110 relative to the subject support 140. This process may be repeated each time a movement of the subject relative to the subject support is identified (and exceeds the predetermined threshold), such that the most-recently generated updated depth map is always used to identify any further movement of the subject. Any further 2D image frame received after an image frame in which movement of the subject has been identified but before the updated depth map has been completed may also be processed using the updated depth map after generation of the updated depth map to identify any movement of the subject occurring during this time; alternatively, these image frames may be processed immediately after receiving, using the most recent available depth map.

In other examples, the processing system 130 may be configured to update the depth map at predetermined intervals. The processing system may do this by repeating, at predetermined intervals during motion of the subject support 140, the steps of receiving, from the camera 120, a further sequence of 2D image frames of the subject acquired during motion of the subject support, and processing the further sequence of 2D image frames and the support motion data 145 to generate an updated depth map for the subject. In these examples, the processing system is configured to identify movement of the subject 110 relative to the subject support using the most-recently generated updated depth map. In other words, while still in the process of generating a current updated depth map, the processing system may use a most-recent previous version of the depth map for identifying movement of the subject; once the current updated depth map is complete, the current updated depth map may then be used for identifying movement of the subject until a new updated depth map has been generated. The updated depth map may be generated in its entirety (i.e. the previous depth map may be replaced with the updated depth map), or the previous depth map may be updated in one or more regions of the depth map to generate the updated depth map.

The speed at which the subject support is moved during acquisition of each further sequence of 2D image frames is used to generate the respective updated depth map. As described above, the speed of the subject support may be obtained by receiving further support motion data representative of motion of the subject support during acquisition of the further sequence of 2D image frames, or by processing the further sequence of 2D image frames (or other 2D image frames acquired at stage of the imaging procedure having a same subject support speed) to determine the speed of the subject support.

The predetermined intervals may depend on the type of imaging procedure. For instance, in a "step and shoot" imaging procedure, in which the subject support is stationary while imaging data is acquired, and is moved relative to the scanning gantry between imaging data acquisitions, a further sequence of 2D image frames may be acquired each time the subject support is moved, and used to generate an updated depth map. In a helical CT imaging procedure, in which imaging data is acquired while the subject support is moved relative to the scanning gantry, the processing system may be configured continuously update the depth map during the diagnostic scan. For instance, each time the generation of an updated depth map is complete, the processing system may use a further sequence of 2D image frames that were acquired during the time taken to generate the updated depth map in order to generate a new updated depth map.

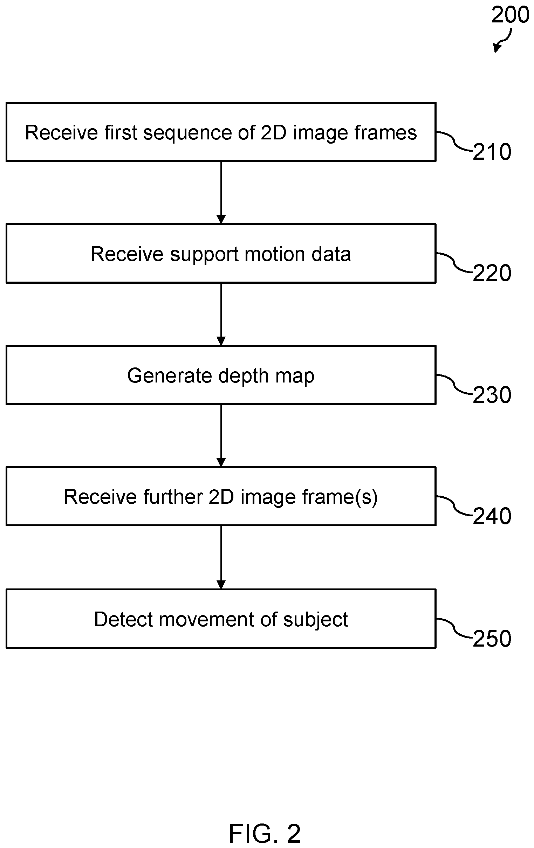

Fig. 2 illustrates a computer-implemented method 200 for detecting movement of a subject during an imaging procedure, according to an embodiment of the invention. The method 200 may be used to detect movement of a subject during any imaging procedure that involves moving a subject support relative to a scanning gantry of a scanner.

The method 200 begins at step 210, at which a first sequence of 2D image frames of the subject is received from a camera. The first sequence of 2D image frames is a sequence of 2D image frames acquired by the camera during a period of motion of the subject support prior to a diagnostic scan of the imaging procedure (e.g. prior to or during a localizer scan of the subject).

At step 220, support motion data representative of motion of the subject support during the acquisition of the first sequence of 2D image frames is received.

At step 230, the first sequence of 2D image frames and the support motion data are processed to generate a depth map for the subject. The generated depth map provides an average depth value for each of a plurality of points on the surface of the subject, adjusted for subject support position.

At step 240, one or more further 2D image frames of the subject acquired by the camera are received.

At step 250, the depth map and the one or more further 2D image frames are processed to identify any movement of the subject relative to the subject support.

It will be understood that the disclosed methods are computer-implemented methods. As such, there is also proposed a concept of a computer program comprising code means for implementing any described method when said program is run on a processing system.

The skilled person would be readily capable of developing a processing system for carrying out any herein described method. Thus, each step of a flow chart may represent a different action performed by a processing system, and may be performed by a respective module of the processing system.

As discussed above, the system makes use of a processing system to perform the data processing. The processing system can be implemented in numerous ways, with software and/or hardware, to perform the various functions required. The processing system typically employs one or more microprocessors that may be programmed using software (e.g. microcode) to perform the required functions. The processing system may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processing system may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processing system or controller may be transportable, such that the one or more programs stored thereon can be loaded into a processing system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processing system may be implemented by a single processing system or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A processing system (130) for detecting movement of a subject (110) during an imaging procedure, wherein the imaging procedure involves moving a subject support (140) relative to a scanning gantry (150) of a scanner, the processing system being configured to:
receive, from a camera (120), a first sequence of 2D image frames (125) of the subject acquired by the camera during a period of motion of the subject support prior to a diagnostic scan of the imaging procedure;
receive support motion data (145) representative of motion of the subject support during the acquisition of the first sequence of 2D image frames;
process the first sequence of 2D image frames and the support motion data to generate a depth map for the subject, wherein the generated depth map provides an average depth value for each of a plurality of points on the surface of the subject, adjusted for subject support position;
receive, from the camera, one or more further 2D image frames of the subject acquired by the camera; and
process the depth map and the one or more further 2D image frames to identify any movement of the subject relative to the subject support.

2. The processing system (130) of claim 1, wherein the processing system is configured to generate the depth map by:
processing the first sequence of 2D image frames (125) and the support motion data to determine a set of 3D coordinates for each 2D image frame in the first sequence;
processing the first sequence of 2D image frames and the support motion data (145) to translate each set of 3D coordinates to a same reference position;
processing each translated set of 3D coordinates to determine an average set of 3D coordinates; and
processing the average set of 3D coordinates to generate the depth map.

3. The processing system (130) of claim 1 or 2, wherein the first sequence of 2D image frames (125) comprises at least two 2D image frames that capture different positions of the subject support (140), wherein the different positions of the subject support captured by the first sequence of 2D image frames encompass a predicted range of positions of the subject support during the diagnostic scan of the imaging procedure.

4. The processing system (130) of any of claims 1 to 3, wherein the first sequence of 2D image frames (125) is acquired by the camera (120) prior to or during a localizer scan of the subject.

5. The processing system (130) of any of claims 1 to 4, wherein the processing system is further configured to process the depth map and the one or more further 2D image frames to identify a region of interest in each of the one or more further 2D image frames.

6. The processing system (130) of claim 5, wherein the processing system is configured to identify any movement of the subject (110) relative to the subject support (140) by processing the depth map and the one or more further 2D image frames to identify any movement of the subject relative to the subject support within the region of interest.

7. The processing system (130) of claim 5 or 6, wherein the processing system is further configured to:
generate an indication of a position of the region of interest in each of the one or more further 2D image frames; and
control a display device (160) to display the indication of the position of the region of interest.

8. The processing system (130) of any of claims 5 to 7, wherein the region of interest defines a position of a scan plane in each of the one or more further 2D image frames.

9. The processing system (130) of any of claims 1 to 8, wherein the processing system is further configured to, in response to identifying a movement of the subject relative to the subject support:
determine whether the identified movement satisfies one or more predetermined conditions; and
in response to a determination that the identified movement satisfies the one or more predetermined conditions, control an output user interface to provide a user-perceptible output representative of the detected movement.

10. The processing system (130) of any of claims 1 to 9, wherein the processing system is further configured to, in response to identifying a movement of the subject relative to the subject support:
determine a measure of movement for the identified movement;
in response to the measure of movement exceeding a predetermined threshold:
receive, from the camera (120), a second sequence of 2D image frames of the subject acquired by the camera during motion of the subject support following the identified movement;
process the second sequence of 2D image frames to generate an updated depth map for the subject; and
for any further 2D image frame received after generating the updated depth map, process the updated depth map and the further 2D image frame to identify any further movement of the subject (110) relative to the subject support (140).

11. The processing system (130) of any of claims 1 to 9, wherein the processing system is further configured to repeat, at predetermined intervals during motion of the subject support (140), the steps of:
receiving, from the camera (120), a further sequence of 2D image frames of the subject acquired by the camera during the motion of the subject support; and
processing the further sequence of 2D image frames to generate an updated depth map for the subject,
wherein the processing system is configured to, for any further 2D image frame received after generating the updated depth map, process the most-recently generated updated depth map and the further 2D image frame to identify any movement of the subject (110) relative to the subject support.

12. The processing system (130) of any of claims 1 to 11, wherein the processing system is further configured to control the scanner based on whether or not movement of the subject relative to the subject support is identified.

13. A system (100) for detecting movement of a subject (110) during an imaging procedure, wherein the imaging procedure involves moving a subject support (140) relative to a scanning gantry (150) of a scanner, the system comprising:
a camera (120) configured to acquire 2D image frames (125) of the subject during the imaging procedure; and
the processing system (130) of any of claims 1 to 12.

14. A computer-implemented method (200) for detecting movement of a subject (110) during an imaging procedure, wherein the imaging procedure involves moving a subject support (140) relative to a scanning gantry (150) of a scanner, the computer-implemented method comprising:
receiving, from a camera (120), a first sequence of 2D image frames (125) of the subject acquired by the camera during a period of motion of the subject support prior to a diagnostic scan of the imaging procedure;
receiving support motion data (145) representative of motion of the subject support during the acquisition of the first sequence of 2D image frames;
processing the first sequence of 2D image frames and the support motion data to generate a depth map for the subject, wherein the generated depth map provides an average depth value for each of a plurality of points on the surface of the subject, adjusted for subject support position;
receiving, from the camera, one or more further 2D image frames of the subject acquired by the camera; and
processing the depth map and the one or more further 2D image frames to identify any movement of the subject relative to the subject support.

15. A computer program product comprising computer program code means which, when executed on a computing device having a processing system, cause the processing system to perform all of the steps of the method (200) according to claim 14.
